# EUROPÄISCHE PATENTANMELDUNG

(11) **EP 2 692 844 A1**
(43) Veröffentlichungstag der Anmeldung: **05.02.2014**
(21) Anmeldenummer: 12178831.9
(22) Anmeldetag: 01.08.2012
(51) Int. Cl.: C11D 3/39, C11D 3/20, C11D 3/06, C11D 1/66, C11D 17/00, C11D 11/00, C11D 3/48, A01N 25/12, A01N 37/26, A01N 43/64, A01N 43/90, A01N 43/707, A01N 37/16, A01N 59/14, A01N 59/00, A01N 25/30, C11D 1/72, C11D 1/00

(54) **Reinigungs- und Desinfektionsmittel für medizinische Instrumente**

(71) Anmelder: Chemische Fabrik Dr. Weigert GmbH & Co KG, 20539 Hamburg (DE)
(72) Erfinder: Strodtholz, Iris, 22765 Hamburg (DE); Schmidt, Verona, 22609 Hamburg (DE); Kamer, Markus, 21465 Reinbek (DE)
(74) Vertreter: Glawe, Delfs, Moll

(57) **Zusammenfassung**

Die Erfindung betrifft ein Reinigungs- und/oder Desinfektionsmittel für medizinische und/oder chirurgische Elemente und Apparate, das als Pulver formuliert ist. Es enthält wenigstens ein Peroxid, wenigstens ein Acylierungsmittel zur Freisetzung von Peressigsäure aus dem Peroxid in wässriger Lösung sowie wenigstens ein nichtionisches Tensid. Erfindungsgemäß ist vorgesehen, dass es Mittel zur Einstellung eines pH-Werts einer 2%igen wässrigen Lösung auf pH 7,5 bis 9 enthält und als Granulat formuliert ist, wobei Peroxid und/oder Acylierungsmittel mit nichtionischem Tensid gecoatet sind.

## Beschreibung

Die Erfindung betrifft ein Reinigungs- und/oder Desinfektionsmittel für medizinische und/oder chirurgische Elemente und Apparate, das als Pulver formuliert ist. Es enthält wenigstens ein Peroxid, wenigstens ein Acylierungsmittel zur Freisetzung von Peressigsäure aus dem Peroxid in wässriger Lösung sowie wenigstens ein nichtionisches Tensid.

Desinfektionsmittel für medizinische und/oder chirurgische Instrumente und Apparate sind in der Praxis weit verbreitet. Die Desinfektionswirkung basiert häufig auf Aldehyden, quartären Armoniumverbindungen, Phenolen, Alkoholen oder anderen Wirkstoffen.

Ebenfalls bekannt sind Reinigungs- und Desinfektionsmittel auf Basis von Peroxiden, insbesondere Peressigsäure. Peroxide sind gut antimikrobiell wirksam, allerdings in der Regel nur wenig lagerstabil.

EP 1 489 908 A1 offenbart bereits ein Mittel gemäß dem Oberbegriff des Anspruchs 1. Gegenüber diesem Stand der Technik liegt der Erfindung die Aufgabe zugrunde, ein Reinigungs- bzw. Desinfektionsmittel der eingangs genannten Art zu schaffen, das eine gute Lagerfähigkeit zeigt und nach dem Auflösen in Wasser schnell sein volles Wirkspektrum entfaltet.

Erfindungsgemäß wird diese Aufgabe dadurch gelöst, dass es Mittel zur Einstellung eines pH-Werts einer 2%igen wässrigen Lösung (alle Angaben im Rahmen der Erfindung sind Gew.-%) auf pH 7,5 bis 9 enthält und als Granulat formuliert ist, wobei Peroxid und/oder Acylierungsmittel mit nichtionischem Tensid gecoatet sind.

Zunächst seien einige im Rahmen der Erfindung verwendete Begriffe erläutert.

Der Begriff Pulver bezeichnet streu- bzw. rieselfähige Feststoffe einschließlich Granulate.

Reinigungs- und/oder Desinfektionsmittel für medizinische und/oder chirurgische Elemente und Apparate sind solche Mittel, die im Zuge der Aufarbeitung solcher Instrumente die Schmutz- und insbesondere Keimbelastung zumindest verringern. Die Erfindung ist besonders geeignet im Zuge der Reinigung bzw. Desinfektion von Endoskopen.

Acylierungsmittel sind solche Verbindungen, die in wässriger Lösung aus dem Peroxid Peressigsäure freisetzen können. Es kann sich beispielsweise um Acyloxycarbonsäuren, insbesondere aber N-Acylverbindungen handeln.

Die Mittel zur Einstellung des erfindungsgemäß vorgesehenen pH-Wertes von 7,5 bis 9 können insbesondere geeignete Säuren und/oder Puffersysteme umfassen. Die übliche Anmeldungskonzentration, bei der sich der pH-Wert einstellen soll, ist eine Lösung von 2 Gew.-% des Pulvers in Wasser. Erfindungsgemäß kann es vorgesehen und bevorzugt sein, dass sich dieser pH-Wert auch bei einer 1 %igen Lösung sowie den dazwischen liegenden Konzentrationen einstellt.

Der Begriff Granulat bezeichnet im Rahmen der Erfindung einen rieselfähigen Feststoff, bei dem Peroxid, Acylierungsmittel, gegebenenfalls auch weitere Bestandteile wie beispielsweise die Mittel zur Einstellung des pH-Wertes mit dem nichtionischen Tensid gecoatet sind. Dies bedeutet, dass jedenfalls ein erheblicher Teil des nichtionischen Tensids im Bereich der Oberflächen der Granulatpartikel angereichert ist. Das Coaten kann durch geeignete und dem Fachmann geläufige Verfahren wie beispielsweise Aufsprühen geschehen. Erfindungsgemäß verwendete nichtionische Tenside sind bei Raumtemperatur oder nach geringfügiger Erwärmung auf bspw. 30 bis 40°C hinreichend flüssig oder fließfähig, um versprüht werden zu können.

Die Korngröße des Granulats kann erfindungsgemäß bevorzugt im Bereich 0,1 bis 2 mm, weiter vorzugsweise 0,2 bis 1,6 mm, weiter vorzugsweise 0,4 bis 1,2 mm betragen. Vorzugsweise mindestens 80 Gew.-%, weiter vorzugsweise mindestens 90 Gew.-% des Granulats fallen dann in den jeweiligen Größenbereich.

Chirurgische Instrumente wie insbesondere Endoskope werden nach der Verwendung häufig zunächst manuell vorgereinigt, bevor sie einem kompletten Aufbereitungszyklus unterworfen werden. Üblich ist häufig eine Vorreinigung unmittelbar im Anschluss an die Verwendung, bei der eine Keimbelastung soweit verringert werden soll, dass eine Gefährdung des dekontaminierte Instrumente handhabenden Personals minimiert wird. Alternativ oder zusätzlich wird häufig auch eine manuelle Desinfektion in einem Tauchbad praktiziert.

In beiden Fällen kommt es darauf an, dass nach dem Ansetzen einer entsprechenden wässrigen Lösung aus dem Feststoff schnell eine antimikrobielle Wirkung eintritt, sich also in der wässrigen Lösung schnell eine wirksame Konzentration freier Peressigsäure einstellt. Die Erfindung hat erkannt, dass durch das Coaten des Granulats mit dem n-Tensid eine schnelle Disintegration des Granulats und der physikalische Löseprozess gefördert wird. Die Einstellung des pH-Wertes auf den beanspruchten Bereich 7,5 bis 9 in der anwendungsfertig verdünnten Lösung trägt dazu bei, dass das Acylierungsmittel schnell hinreichende Mengen Peressigsäure aus dem Peroxid freisetzt und so spätestens nach 15 min eine für die Anwendungszwecke hinreichende und damit wirksame Peressigsäurekonzentration in der wässrigen Lösung vorhanden ist. Besonders bevorzugt ist erfindungsgemäß die Einstellung des pH-Wertes auf den Bereich 7,5 bis 8,5, weiter bevorzugt 7,6 bis 7,9.

Das Coaten des Granulats mit dem n-Tensid trägt ferner dazu bei, dass die bei der Lagerung eines Gemisches von Acylierungsmitteln und Peroxiden stattfindende Zersetzungsreaktion minimiert wird und somit die Lagerfähigkeit des erfindungsgemäßen Mittels erhöht wird.

Die Erfindung stellt somit ein bei üblicher Lagerung bei Raumtemperatur, vorzugsweise auch bei 40°C wenigstens ein Jahr, vorzugsweise wenigstens zwei Jahre lagerfähiges Mittel zur Verfügung, dass nach Auflösen in Wasser schnell (binnen 15 min) eine wirksame Konzentration freier Peressigsäure zur Verfügung stellt. Typische Anwendungskonzentrationen eines erfindungsgemäßen Mittels in wässriger Lösung sind 1 oder 2 Gew.-%. In einer 1%igen Lösung stellt sich nach 15 min typischerweise eine Konzentration freier Peressigsäure von wenigstens 900 ppm, typischerweise ca. 1.500 ppm ein. Bei einer 2%igen Lösung sind es mindestens 2.600 ppm, typischerweise 3.000 ppm.

Als Peroxide werden erfindungsgemäß bevorzugt anorganische Peroxide wie insbesondere Perborate oder Percarbonate eingesetzt. Bevorzugt sind die Natriumsalze. Besonders bevorzugt ist Natriumpercarbonat. Erfindungsgemäß ist es möglich und bevorzugt, dass das eingesetzte Peroxid vor dem Vermischen ebenfalls mit einem Coating versehen wird, um unerwünschte Zersetzungsreaktionen bei der Lagerung gemeinsam mit dem Acylierungsmittel zu minimieren. Beispielsweise kann das Peroxid mit einem Oleat-Natriumsulfat-Coating versehen sein.

Das Acylierungsmittel kann erfindungsgemäß ausgewählt sein aus der Gruppe bestehend aus Tetraacetylethylendiamin (TAED), Tetraacetylglykoluril und Diacetylhexahydrotriazindion. Tetraacethylendiamin (TAED) ist besonders bevorzugt.

Die nichtionischen Tenside können erfindungsgemäß ausgewählt sein aus der Gruppe bestehend aus Fettalkoholethoxylaten, Fettalkoholpropoxylaten, EO-PO-Blockcopolymeren, Alkylglucosiden, Alkylpolyglucosiden, Oktylphenolethoxylaten und Nonylphenolethoxylaten. Dem Fachmann geläufige ethoxylierte Fettalkohole sind besonders bevorzugt.

Als Mittel zur Einstellung des pH-Werts sind bevorzugt Säuren und/oder ein Puffersystem vorgesehen. Besonders bevorzugt sind Citronensäure (bevorzugt das wasserfreie Anhydrid), Citratpuffer, Phosphat- oder Carbonatpuffer. Erfindungsgemäß kann beispielsweise Citronensäure mit einem Phosphat, bevorzugt Natriumtripolyphosphat, kombiniert werden.

Das erfindungsgemäße Mittel ist als Feststoff formuliert. Bevorzugt ist es im wesentlichen wasserfrei, um Zersetzungsreaktionen des Peroxids zu minimieren und die Lagerstabilität zu erhöhen. Die Summe der Masseanteile von Peroxid, Acylierungsmittel und Mittel zur Einstellung des pH-Werts macht bevorzugt wenigstens 60 Gew.-%, weiter vorzugsweise wenigstens 70 Gew.-%, weiter vorzugsweise wenigstens 80 Gew.-%, weiter vorzugsweise wenigstens 90 Gew.-% der Gesamtmasse des erfindungsgemäßen Mittels aus. Diese Summe kann beispielsweise 95 Gew.-% der Gesamtmasse des Mittels ausmachen.

Bevorzugt sind im Rahmen der Erfindung folgende Gewichtsanteile der Inhaltsstoffe bezogen auf die Gesamtmasse des als Feststoff formulierten Mittels:
- Peroxid 40 - 70, vorzugsweise 50 - 60 Gew.-%;
- Acylierungsmittel 15 - 40, vorzugsweise 20 - 30 Gew.-%,
- Mittel zur Einstellung des pH-Werts 5 - 25 Gew.-%, vorzugsweise 10 - 20 Gew.-%,
- nichtionisches Tensid 1 - 5 Gew.-%, vorzugsweise 2 - 4 Gew.-%.

Gegenstand der Erfindung ist ferner ein Verfahren zur Herstellung eines erfindungsgemäßen Mittels. Bei diesem Verfahren werden zunächst Peroxid, Acylierungsmittel und Mittel zur Einstellung des pH-Werts (gegebenenfalls auch weitere fakultative Inhaltsstoffe) miteinander vermischt. Anschließend wird unter Aufsprühen des flüssigen n-Tensids granuliert. Das Coaten kann beispielsweise mittels eines Rotationssprühmischers geschehen. Erfindungsgemäß verwendbare typische Sprühparameter sind ein Sprühdruck von 4 bar und eine Sprühleistung von 175 g/s.

Bei dem oben erwähnten fakultativen weiteren Zusatzstoffen kann es sich beispielsweise um Enzyme, insbesondere proteolytische Enzyme, weitere Tenside oder dergleichen handeln. Diese Inhaltsstoffe können dazu beitragen, die Reinigungsleistung des erfindungsgemäßen Mittels zu verbessern.

Gegenstand der Erfindung ist ferner ein Verfahren zur Reinigung und Desinfektion von medizinischen und/oder chirurgischen Instrumente und/oder Apparaten (insbesondere Endoskopen), das folgende Schritte umfasst:
a) Herstellen einer 0,5 bis 3%igen, vorzugsweise 1 bis 2%igen wässrigen Lösung eines Reinigungs-und/oder Desinfektionsmittel nach einem der Ansprüche 1 bis 9,
b) Reinigen und/oder Desinfizieren der medizinischen und/oder chirurgischen Instrumente und Apparate.

Die Anwendungskonzentration kann abhängig vom vorgesehenen Verwendungszweck sein. Will man lediglich eine Vorreinigung zur Verminderung der Keimbelastung durchführen, kann beispielsweise eine Desinfektion in einer 1 %igen Lösung über einen Zeitraum von 5 min ausreichend sein. Soll eine vollständige Reinigung und Desinfektion (vorzugsweise manuell) erfolgen, wird erfindungsgemäß bevorzugt beispielsweise ein 2 %iges Tauchbad gewählt über einen Zeitraum von 15 min gereinigt und desinfiziert.

Dies erfolgt erfindungsgemäß bevorzugt im Tauchbad.

Bei manueller Anwendung liegt die Temperatur der Lösung bei der Anwendung bevorzugt bei etwa 20° C (Raumtemperatur) bis 40° C.

Die Reinigung bzw. Desinfektion erfolgt bevorzugt über einen Zeitraum von 5 bis 30 min, weiter vorzugsweise 10 bis 20 min.

Ausführungsbeispiele der Erfindung werden nachfolgend beschrieben.

### Beispiel 1

Zur Herstellung eines erfindungsgemäßen Reinigungs- und Desinfektionsmittels werden folgende Ausgangsmaterialien in den angegebenen Gewichtsanteilen eingesetzt:

| | Gew.-% |
|---|---|
| Na-Percarbonat (Fa. Harke Chemicals) | 55,0 |
| TAED (Peractive® P, Fa. Clariant) | 25,0 |
| Na-Tripolyphosphat (Sackware, grob 850 - 1100, Fa. BK Giulini) | 2, 0 |
| Citronensäure anhydr. | 15,0 |
| PPG-4 Laureth-5 (Fettalkohol C10/12, 5 EO/4 PO, Fa. Cognis) | 3, 0 |

Die Pulverbestandteile (sämtliche Bestandteile bis auf das n-Tensid) werden unter Rühren in einen Rotationsmischer gegeben und gleichmäßig vermischt. Das n-Tensid wird auf 40°C erwärmt und binnen 4 min bei einem Sprühdruck von 4 bar auf die Pulvermischung unter fortlufendem weiteren Mischen aufgesprüht. Anschließend wird weiter für etwa 12 min gemischt.

### Beispiel 2

Es wird wie in Beispiel 1 verfahren, die eingesetzten Ausgangsmaterialien sind die folgenden:

| | Gew.-% |
|---|---|
| Na-Percarbonat (Fa. Harke Chemicals) | 49,0 |
| TAED (Peractive® AN, Peroxid mit einem Oleat-Natriumsulfat-Coating, Fa. Clariant) | 29,0 |
| Na-Tripolyphosphat (Sackware, grob 850 - 1100, Fa. BK Giulini) | 4, 0 |
| Citronensäure anhydr. | 12, 0 |
| Dehypon® GRA (modifizierter Fettalkoholpolyglykolether, Fa. BASF) | 4, 0 |
| K-Carbonat (wasserfrei, ca. 95%ig) | 2, 0 |

In diesem Beispiel wird ein anderes n-Tensid eingesetzt und das Puffersystem durch den Einsatz von Kaliumcarbonat modifiziert.

### Beispiel 3

Es wird wie in Beispiel 1 verfahren, die eingesetzten Ausgangsmaterialien sind die folgenden:

| | Gew.-% |
|---|---|
| Na-Percarbonat (Fa. Harke Chemicals) | 50,0 |
| TAED (Peractive® AN, Peroxid mit einem Oleat-Natriumsulfat-Coating, Fa. Clariant) | 29,0 |
| Na-Tripolyphosphat (Sackware, grob 850 - 1100, Fa. BK Giulini) | 3, 0 |
| Citronensäure anhydr. | 15,0 |
| Fettalkohol C10, 11 EO (Lutensol® ON110, Fa. BASF) | 3, 0 |

In diesem Beispiel wird ein weiteres n-Tensid eingesetzt.

### Beispiel 4

Es wird wie in Beispiel 1 verfahren, die eingesetzten Ausgangsmaterialien sind die folgenden:

| | Gew.-% |
|---|---|
| Na-Percarbonat (Fa. Harke Chemicals) | 50,0 |
| TAED (Peractive® AN, Peroxid mit einem Oleat-Natriumsulfat-Coating, Fa. Clariant) | 29,0 |
| Esperase (Fa. Novozymes) | 1,0 |
| Citronensäure anhydr. | 15,0 |
| Fettalkohol C10/12, !0 EO (Dehypon® LS 104 l, Fa. BASF) | 2, 0 |
| K-Carbonat (wasserfrei, ca. 95%ig) | 3, 0 |

In diesem Beispiel wird wiederum ein anderes n-Tensid eingesetzt, ferner ist zusätzlich ein proteolytisches Enzym enthalten.

### Beispiel 5

In diesem Beispiel wird eine Vorreinigung bzw. Vordesinfektion (teilweise Desinfektion dahingehend, dass die Kontaminationsgefahr für Personen, die die Endoskope anschließend handhaben, minimiert wird) durchgeführt.

In einer Reinigungswanne werden 1 Gew._% des Beispiels 1 in lauwarmem Wasser gelöst. Nach 15 min hat sich eine Konzentration von ca. 1.500 ppm freie Peressigsäure eingestellt. Erfindungsgemäß sollte diese Konzentration möglichst bei 900 ppm oder darüber liegen.

Direkt im Anschluss an die Untersuchung word noch im Untersuchungsraum das Endoskop (das noch an der Lichtquelle und der Absaugpumpe angeschlossen ist) mit einem flusenfreien Tuch abgewischt. Es soll ein Antrocknen der organischen Rückstände vermieden werden. Das Endoskop wird dann in die Lösung getaucht und alle Kanäle werden mehrfach durchgespült bzw durchgesaugt. Das Endoskop wird von der Lichtquelle und der Absauganlage getrennt, die Reinigungswanne verschlossen und in den Aufbereitungsraum transportiert.

### Beispiel 6

In diesem Beispiel wird eine manuelle Reinigung und Desinfektion von Endoskopen durchgeführt.

In einer Reinigungswanne werden 2 Gew._% des Beispiels 1 in lauwarmen Wasser gelöst. Nach 15 min hat sich eine Konzentration von ca. 3000 ppm freie Peressigsäure eingestellt. Das Endoskop oder die Zusatzinstrumente werden so in die Lösung gelegt, dass alle Oberflächen vollständig benetzt sind und keine Luftblasen vorhanden sind. Alle Reinigungsschritte finden unterhalb der Flüssigkeitsoberfläche statt. Die Endoskopkanäle werden sorgfältig mit Bürsten gereinigt, wobei für jeden Kanal eine passende desinfizierte Bürste verwendet wird unter Beachtung von Angaben des Endoskopherstellers. Zur Abspülung der Anwendungslösung ist jeweils frisches, mikrobiologisch einwandfreies Wasser zu verwenden, wobei alle Kanäle und der Außenmantel des Endoskops sorgfältig gespült werden müssen

### Beispiel 7

Für eine wirksame Desinfektion ist es von Bedeutung, dass sich nach dem Ansetzen der Lösung aus dem erfindungsgemäßen Mittel schnell eine hinreichende Konzentration freier Peressigsäure einstellt und über einen längeren Anwendungszeitraum in der Lösung erhalten bleibt.

Es wird eine Lösung von 2 Gew.-% des Mittels des Beispiels 1 in Stadtwasser hergestellt (15 min Rühren bei 25°C). Die Konzentration der freien Peressigsäure in der Lösung wird bestimmt, die Bestimmung wird wiederholt in den in der Tab. 2 angegebenen Zeitabständen. Man erkennt, dass sich unmittelbar nach dem Ansetzen der Lösung eine wirksame Konzentration einstellt und über einen üblichen Anwendungszeitraum von 8 h erhalten bleibt.

**Tab. 2**

| Zeit [h] | Peressigsäure [ppm] |
|---|---|
| 0 | 3.345 |
| 1 | 2.991 |
| 2 | 2.631 |
| 3 | 2.564 |
| 4 | 2.354 |
| 5 | 2.106 |
| 6 | 2.054 |
| 7 | 1.880 |
| 8 | 1.591 |

### Beispiel 8

Zur Überprüfung der Lagerfähigkeit des erfindungsgemäßen Mittels werden jeweils 2 kg Pulver des Beispiels 1 in einen 3 l Eimer (Polypropylen) gefüllt und der Eimer mit einem Deckel verschlossen. Die so hergestellten Muster wurden über einen Zeitraum von insgesamt zwölf Wochen unterschiedlichen Lagerungsbedingungen wie folgt ausgesetzt:
- 6°C, 55% rel. Feuchte
- 21°C, 55% rel. Feuchte
- 30°C, 70% rel. Feuchte
- 40°C, 75% rel. Feuchte
- Wechselklima 6°C, 21°C, 40°C, 21°C, 6°C jeweils 5h mit jeweils 1 h Übergangszeit zwischen den Temperaturen

Jedes Muster wurde nach 4, 8 und 12 Wochen Lagerung auf Aussehen von Pulver bzw. Granulat und daraus hergestellte 2%iger wässriger Lösung, pH-Wert der Lösung und Konzentration von freier Peressigsäure in der Lösung untersucht.

Bei sämtlichen Mustern und sämtlichen Lagerungsbedingungen ergab sich waren die Ergebnisse wie folgt:
Das Granulat behielt seine ursprüngliche weiß-gelbliche Farbe und Rieselfähigkeit. Die wässrige Lösung war klar und farblos. Der Gehalt an freier Peressigsäure in der wässrigen Lösung nach 15 min Rühren betrug zwischen 2.600 und 3.000 ppm. Der pH-Wert der Lösung lag zwischen 7,6 und 7,8.

Diese Lagerungsversuche erlauben den Schluss, das ein erfindungsgemäßes Mittel bei Raumtemperatur wenigstens 2 Jahre lagerfähig ist.

### Beispiel 9

In diesem Beispiel wird die Reinigungswirkung eines erfindungsgemäßen Mittels untersucht. Ein primäres Ziel der Erfindung ist es, durch das Freisetzen freier Peressigsäure in der wässrigen Lösung desinfizierend zu wirken. Die umfassende und gute Desinfektionswirkung von Peressigsäure ist im Stand der Technik seit langem bekannt.

Zusätzlich soll das erfindungsgemäße Mittel vorteilhafter Weise zu einer Reinigung ebenfalls beitragen. Die nachfolgend durchgeführten Tauchversuche sollen diese Reinigungsleistung quantifizieren. Der Versuch ist so angelegt (bloßes Eintauchen in die Lösung ohne unmittelbare mechanische Einwirkung auf die verschmutzten Flächen) dass eine vollständige Entfernung der Testanschmutzung nicht möglich ist und somit auch nicht erwartet werden kann.

Als Schmutzträger wurden raue Metallplättchen 5 x 10 cm verwendet. Auf die gereinigten und gewogenen Metallplättchen wurden 200 µl defibriniertes Schafblut (Fa Acila Ch. B. 24632), aufgetragen und über Nacht bei Raumtemperatur getrocknet. Anschließend wurden die Prüfplättchen ausgewogen.

In einem 600 ml Becherglas wurden 500 g 2%ige wässrige Lösung des Beispiels 1 bereitgestellt. Die Lösung wurde während der Versuchsdurchführung bei Raumtemperatur mit einem Magnetrührer (Stufe 8) gerührt.

Die Prüfplättchen wurden 15 min in die Lösung eingetaucht und anschließend kurz durch Eintauchen in VE-Wasser gespült. Nach einem Trocknen über Nacht wurden die Prüfplättchen erneut ausgewogen.

Von der Testanschmutzung verblieb nach diesem Versuch eine Restmenge von 48,6 Gew.-% auf den Prüfplättchen. Bei zum Vergleich geprüften handelsüblichen Desinfektionsmitteln für Endoskope lag die Restmenge deutlich darüber (bis zu 96,9 Gew.-%). Dies zeigt, dass das erfindungsgemäße Mittel nicht nur eine gute Desinfektion bewirkt, sondern auch zur Reinigung maßgeblich beiträgt.

## Patentansprüche

1. Als Pulver formuliertes Reinigungs- und/oder Desinfektionsmittel für medizinische und/oder chirurgische Instrumente und Apparate, das enthält:
a) wenigstens ein Peroxid,
b) wenigstens ein Acylierungsmittel zur Freisetzung von Peressigsäure aus dem Peroxid in wässriger Lösung,
c) wenigstens ein nichtionisches Tensid, **gekennzeichnet durch** folgende Merkmale:
d) es enthält Mittel zur Einstellung eines pH-Werts einer 2 %igen wässrigen Lösung auf pH 7,5 bis 9,
e) es ist als Granulat formuliert, wobei Peroxid und/oder Acylierungsmittel mit nichtionischem Tensid gecoatet sind.

2. Reinigungs- und/oder Desinfektionsmittel nach Anspruch 1, **dadurch gekennzeichnet, dass** das Peroxid ein Perborat und/oder Percarbonat umfasst.

3. Reinigungs- und/oder Desinfektionsmittel nach Anspruch 1 oder 2, **dadurch gekennzeichnet, dass** das Acylierungsmittel ausgewählt ist aus der Gruppe bestehend aus Tetraacetylethylendiamin (TAED), Tetraacetylglykoluril und Diacetylhexahydrotriazindion.

4. Reinigungs- und/oder Desinfektionsmittel nach einem der Ansprüche 1 bis 3, **dadurch gekennzeichnet, dass** die nichtionischen Tenside ausgewählt sind aus der Gruppe bestehend aus Fettalkoholethoxylaten, Fettalkoholpropoxylaten, EO-PO-Blockcopolymeren, Alkylglucosiden, Alkylpolyglucosiden, Oktylphenolethoxylaten und Nonylphenolethoxylaten.

5. Reinigungs- und/oder Desinfektionsmittel nach einem der Ansprüche 1 bis 4, **dadurch gekennzeichnet, dass** es als Mittel zur Einstellung des pH-Werts eine Säure und/oder ein Puffersystem enthält.

6. Reinigungs- und/oder Desinfektionsmittel nach Anspruch 5, **dadurch gekennzeichnet, dass** es als Mittel zur Einstellung des pH-Werts Citronensäure und/oder einen Phosphat- oder Carbonatpuffer enthält.

7. Reinigungs- und/oder Desinfektionsmittel nach Anspruch 6, **dadurch gekennzeichnet, dass** es als Phosphatpuffer Natriumtripolyphosphat umfasst.

8. Reinigungs- und/oder Desinfektionsmittel nach einem der Ansprüche 1 bis 7, **dadurch gekennzeichnet, dass** die Summe aus Peroxid, Acylierungsmittel und Mittel zur Einstellung des pH-Werts wenigstens 60 Gew.-%, weiter vorzugsweise wenigstens 70 Gew.-%, weiter vorzugsweise wenigstens 80 Gew.-%, weiter vorzugsweise wenigstens 90 Gew.-% der Gesamtmasse des Reinigungs- und/oder Desinfektionsmittels beträgt.

9. Reinigungs- und/oder Desinfektionsmittel nach einem der Ansprüche 1 bis 8, **gekennzeichnet durch** folgende Gewichtsanteile der Inhaltsstoffe:
- Peroxid 40-70, vorzugsweise 50-60 Gew.-%;
- Acylierungsmittel 15-40, vorzugsweise 20-30 Gew.-%,
- Mittel zur Einstellung des pH-Werts 5-25 Gew.-%, vorzugsweise 10 - 20 Gew.-%,
- nichtionisches Tensid 1-5 Gew.-%, vorzugsweise 2-4 Gew.-%.

10. Verfahren zur Herstellung eines Reinigungs-und/oder Desinfektionsmittel nach einem der Ansprüche 1 bis 9, **gekennzeichnet durch** folgende Schritte:
a) Vermischen von Peroxid, Acylierungsmittel und Mittel zur Einstellung des pH-Werts,
b) Durchführen einer Granulierung unter Aufsprühen des nichtionischen Tensids.

11. Verfahren zur Reinigung und/oder Desinfektion von medizinischen und/oder chirurgischen Instrumenten und Apparaten, **gekennzeichnet durch** folgende Schritte:
a) Herstellen einer 0,5 bis 3%igen, vorzugsweise 1 bis 2%igen wässrigen Lösung eines Reinigungs-und/oder Desinfektionsmittel nach einem der Ansprüche 1 bis 9,
b) Reinigen und/oder Desinfizieren der medizinischen und/oder chirurgischen Instrumente und Apparate.

12. Verfahren nach Anspruch 11, **dadurch gekennzeichnet, dass** die Reinigung/Desinfektion im Tauchbad erfolgt.

13. Verfahren nach Anspruch 11 oder 12, **dadurch gekennzeichnet, dass** die Reinigung/Desinfektion bei 20 bis 40°C erfolgt.

14. Verfahren nach einem der Ansprüche 11 bis 13,
**dadurch gekennzeichnet, dass** die Reinigung/Desinfektion über einen Zeitraum von 5 bis 30 min, vorzugsweise 10 bis 20 min erfolgt.

15. Verfahren nach einem der Ansprüche 11 bis 14,
**dadurch gekennzeichnet, dass** die Reinigung/Desinfektion manuell erfolgt.
